# EUROPÄISCHE PATENTSCHRIFT

(11) **EP 0 910 665 B1**
(45) Veröffentlichungstag und Bekanntmachung des Hinweises auf die Patenterteilung: **26.06.2002**
(21) Anmeldenummer: 97923750.0
(22) Anmeldetag: 23.04.1997
(51) Int. Cl.: C12Q 1/68

(54) **NACHWEIS NUMERISCHER VERÄNDERUNGEN IN DER DNA VON ZELLEN**
IDENTIFICATION OF NUMERICAL CHANGES IN CELL DNA
DETECTION DE VARIATIONS NUMERIQUES DANS L'ADN DE CELLULES

(30) Priorität: 24.04.1996 DE 19616381
(43) Veröffentlichungstag der Anmeldung: 28.04.1999
(73) Patentinhaber: Deutsches Krebsforschungszentrum Stiftung des öffentlichen Rechts, 69120 Heidelberg (DE)
(72) Erfinder: JOOS, Stefan, D-69221 Dossenheim (DE); LICHTER, Peter, D-69251 Gaiberg (DE)
(74) Vertreter: Schüssler, Andrea, Dr.
(86) Internationale Anmeldenummer: DE9700814
(87) Internationale Veröffentlichungsnummer: WO9740185

(56) Entgegenhaltungen:
- EP-A- 0 341 491
- WO-A-95/14085
- SCIENCE, Band 258, ver!ffentlicht 1992, 30 Oktober A. KALLIONIEMI et al. "Comparative Genomic Hybri- dization for Molecular Cyto- genetic Analysis od Solid Tumors", Seiten 818-821,

## Beschreibung

Die Erfindung betrifft ein Verfahren zum Nachweis numerischer Veränderungen in der DNA von Zellen und einen zur Durchführung des Verfahrens geeigneten Kit.

Im Bereich der molekularen Tumorgenetik ist es wichtig zu wissen, welche genetischen bzw. zytogenetischen Veränderungen in bestimmten Tumoren vorliegen, in welcher Abfolge sie entstehen und ob sie mit dem klinischen Verlauf, z.B. bei einer Therapie, korreliert sind. Um dies erfahren zu können, wäre es notwendig, die DNA kleiner Zellpopulationen bzw. von Einzelzellen der Tumoren untersuchen zu können. Viele Versuche wurden unternommen, dies zu erreichen. Bisherige Ergebnisse sind allerdings nicht befriedigend.

Der vorliegenden Erfindung liegt somit die Aufgabe zugrunde, ein Verfahren bereitzustellen, mit dem genetische bzw. zytogenetische Veränderungen in der DNA kleiner Zellpopulationen bzw. von Einzelzellen nachgewiesen werden können.

Erfindungsgemäß wird dies durch die Gegenstände in den Patentansprüchen erreicht.

Gegenstand der vorliegenden Erfindung ist somit ein Verfahren zum Nachweis numerischer Veränderungen in der DNA von Zellen, das die folgenden Schritte umfaßt:
(a) Isolierung von DNA aus Zellen, die keine bekannten numerischen Veränderungen in ihrer DNA aufweisen, und Amplifikation der DNA mittels eines PCR-Verfahrens unter Verwendung von tag-Primern,
(b) In situ Hybridisierung von zu untersuchenden Zellen mit der amplifizierten DNA von (a),
(c) Amplifikation von DNA aus den in situ hybridisierten Zellen von (b) mittels eines PCR-Verfahrens unter Verwendung der tag-Primer von (a), und
(d) Identifizierung numerischer Veränderungen in der amplifizierten DNA von (c).

Aus solchen Zellen wird DNA isoliert und amplifiziert. Hierfür können übliche Verfahren verwendet werden. Für die Amplifikation bietet sich ein PCR-Verfahren an, bei dem tag-Primer verwendet werden. Der Ausdruck "tag" weist darauf hin, daß die Primer degenerative (universelle) Primer sein können, d.h. es handelt sich um Primer, die an vielen verschiedenen Stellen einer Zell-DNA binden können. Beispiele solcher Primer sind DOP- oder SiA-Primer.

Der Ausdruck "zu untersuchende Zellen" umfaßt Zellen jeglicher Art und Abstammung. Insbesondere sind es Tumorzellen oder Zellen aus dem Blut von schwangeren Personen. Besonders bevorzugt sind Zellen einer kleinen Zellpopulation oder Einzelzellen. Ganz besonders bevorzugt sind Zellen mit einem Interphase-Kern.

Die zu untersuchenden Zellen werden einer in situ Hybridisierung mit der amplifizierten DNA aus den gesunden Zellen unterzogen. Hierfür können übliche Bedingungen und Materialien gewählt werden.

Die DNA aus den in situ hybridisierten Zellen wird als Template für eine Amplifikation verwendet. Hierfür bietet sich ein PCR-Verfahren an, bei dem die vorstehenden tag-Primer entsprechend verwendet werden.

Die amplifizierte DNA wird verwendet, um numerische Veränderungen festzustellen. Hierzu können übliche Verfahren durchgeführt werden. Günstig ist es, ein "comparative genomic hybridization"-Verfahren (CGH) durchzuführen (vgl. Kallioniemi A, et al., Science 258:818, 1992, Kallioniemi O-P, et al., Genes Chromosom Cancer, 10:231, 1994 und Lichter P, et al. in Human Chromosomes, Hrsg. Verma R.S. und Babu A., New York, 1995, S. 191).

Erfindungsgemäß wird auch ein Kit zur Durchführung des Verfahrens zum Nachweis numerischer Veränderungen in der DNA von Zellen, insbesondere von einer kleinen Zellpopulation oder Einzelzellen, bereitgestellt. Ein solcher Kit umfaßt folgende Komponenten:
(a) amplifizierte DNA aus Zellen, die keine bekannten numerischen Veränderungen in ihrer DNA aufweisen, wobei die DNA von tag-Primern flankiert ist,
(b) tag-Primer, und
(c) Hilfsmittel, für die Identifizierung numerischer Veränderungen in einer DNA.

Mit der vorliegenden Erfindung ist es möglich, numerische Veränderungen in der DNA von Zellen, insbesondere kleiner Zellpopulationen und von Einzelzellen, nachzuweisen. Damit eignet sich die vorliegende Erfindung, in der Diagnostik von Erkrankungen eingesetzt zu werden, in denen es auf Untersuchungen kleinster Gewebeproben und Zellverbände ankommt. Solche Erkrankungen sind insbesondere Tumorerkrankungen. Desweiteren eignet sich die vorliegende Erfindung, embryonale Zellen im Blut von schwangeren Personen zu untersuchen. Sie stellt damit eine neue Möglichkeit in der pränatalen Diagnostik dar.

Kurze Beschreibung der Zeichnungen:
- Figur 1: zeigt die schematische Darstellung des erfindungsgemäßen Verfahrens und
- Figur 2: zeigt chromosomale Überrepräsentationen in der Zellinie Colo 320HSR mit und ohne Anwendung des erfindungsgemäßen Verfahrens "Tagged Genome Hybridization" (TGH).

Das folgende Beispiel erläutert die Erfindung.

### Beispiel: Analyse kleiner Zellpopulationen der Zellinie Colo320 (HSR) mit dem erfindungsgemäßen Verfahren

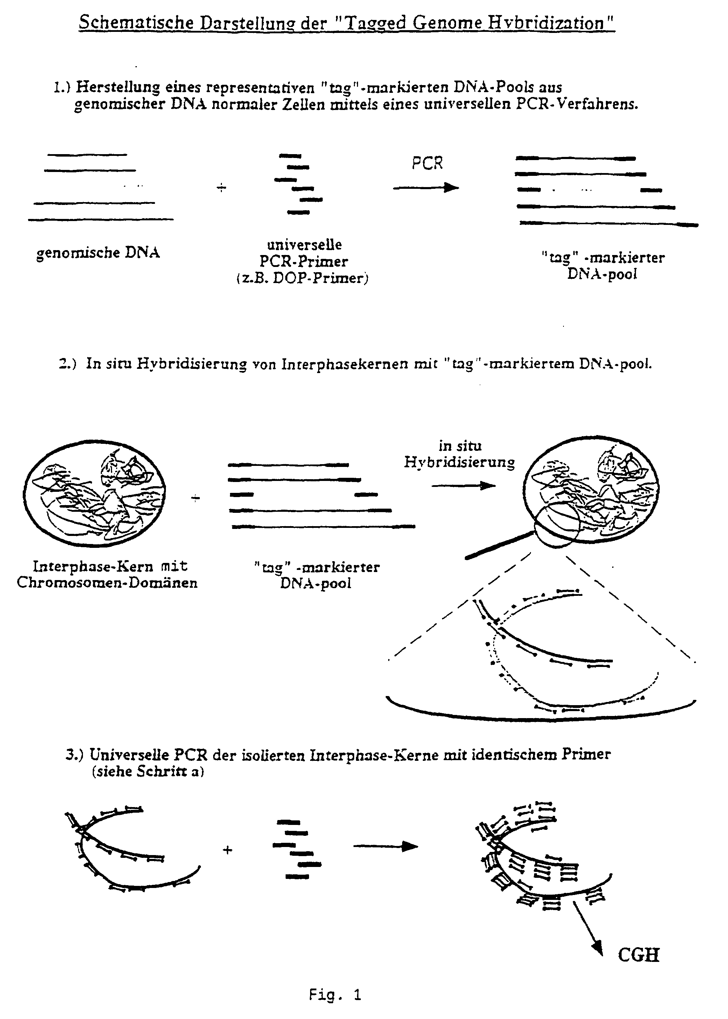

Im folgenden wird das erfindungsgemäße Verfahren (TGH-Verfahren, vgl. Fig. 1) an der Zellinie Colo320 (HSR) durchgeführt. Das TGH-Verfahren verläuft in mehreren Einzelschritten:
- Präparation von Colo320 (HSR)-Interphasekernen auf Objektträger.
- Herstellung eines "tag"-markierten Sequenzpools normaler genomischer DNA mittels DOP-PCR.
- Modifizierung der "tag"-markierten genomischen DNA
- In situ Hybridisierung der "tag"-markierten genomischen DNA auf Interphasekerne der Zellinie Colo320 (HSR).
- Isolierung von Colo320 (HSR)-Zellpopulationen definierter Anzahl mittels Mikromanipulation. Zur Verdeutlichung der Effizienz der TGH-Behandlung wurden sowohl mit "tag"-markierter DNA hybridisierte als auch nichthybridisierte Zellkerne isoliert und im weiteren verglichen.
- DNA-Amplifikation der isolierten Zellkerne mittels DOP-PCR.
- Verfahren der "comparative genomic hybridization" (CGH) zum Nachweis chromosomaler Imbalancen der Colo320 (HSR)-Zellen.

Bezüglich der Präparation der Interphasekerne, der Isolation genomischer DNA aus Blut und des CGH-Protokolls wird auf bekannte Verfahren verwiesen (vgl. Kallioniemi A, et al., Science 258:818, 1992, Kallioniemi O-P, et al., Genes Chromosom Cancer, 10:231, 1994 und Lichter P, et al. in Human Chromosomes, Hrsg. Verma R.S. und Babu A., New York, 1995, S. 191).

### Schritt 1

**Herstellung eines "tag"-markierten Sequenzpools normaler genomischer DNA mittels DOP-PCR.**

### Reagenzien, Puffer und Lösungen:

- 10x Reaktionspuffer: 20 mM MgCl₂; 500 mM KCl; 100 mM Tris-HCl (pH 8.4); 1 mg/ml Gelatine.
- 10x Nukleotid-Mix: 2 mM jedes Nukleotids (dATP, dCTP, dGTP, dTTP) (hergestellt aus dem Deoxynucleoside-Triphosphat-Set von Boehringer Mannheim, Kat.- Nr. 1277049).
- 10x DOP-Primer: 20 µM des Oligonukleotids 5'CCG ACT CGA GNN NNN NAT GTG G 3', (N = A, C, G oder T).
- Für Minigele: Agarose; TBE Puffer: 0,089 M Tris-Borat, 0,089 M Borsäure, 2 mM EDTA (pH 8,0); geeigneter Größenmarker (z.B. 1kb-Marker; Kat.- Nr. 15615-016; Gibco BRL, Eggenstein); Gelladungspuffer mit 0.25% Bromphenolblau und 30% Gycerol.
- Zu markierende bzw. amplifizierende Ausgangs-DNA normaler Zellen.

### Detaillierter Ablauf:

1. In ein 500 µl Reaktionsgefäß (PCR-Gefäße mit integrierter Volumenbegrenzung; Sarstedt, Nümbrecht) wurden auf Eis folgende Komponenten pipettiert (Ges.-Vol. 50 µl):
   100 ng genomische Ausgangs DNA
   5 µl 10x Reaktionspuffer
   5µl 10x Nukleotid-Mix
   5 µl 10x DOP-Primer
   H₂O ad 50 µl
   1.25 U Taq-Polymerase (sollte zuletzt zugesetzt werden).
2. Als Negativkontrolle wurden dieselben Komponenten pipettiert, jedoch ohne Zugabe der Ausgangs-DNA.
3. Für die PCR-Reaktionen kam ein Gerät der Omnigene/Hybaid (Tyg AZ 1623; Lieferant MWG Biotech, Ebersbach b. München) zum Einsatz. Das folgende Temperaturprogramm wurde verwendet:
   - Initiale Denaturierung der Ausgangs DNA durch Erhitzen auf 93°C für 10 min.
   - 5 Zyklen (sog. "low stringency Phase") mit jeweils:
      94°C, 1 min; 30°C, 3 min; 30°C -72°C, 3 min.; 72°C, 3 min.
   - 35 Zyklen (sog. "high stringency Phase") mit jeweils:
      94°C, 1 min.; 62°C, 1 min.; 72°C, 3 min (mit einer Verlängerung von 1 sec /Zyklus);
   - Ein finaler Extensionsschritt bei 72°C für 10 min.
4. Nach der PCR-Reaktion wurde ein Aliquot von 7 µl des Produkts auf einem Agarosegel aufgetrennt. Als Größenmarker diente ein 1-kb Marker. Die Auftrennung erfolgte in 1x TBE-Puffer für 30 min bei 100 Volt. Die aufgetragene DNA wurde durch Anfärbung mit Ethidiumbromid sichtbar gemacht und photographiert. Ein "Schmier" mit DNA-Fragmenten zwischen 200 Basenpaaren und 2000 Basenpaaren war i. d. R. sichtbar. In der Negativkontrolle war keine DNA in diesem Größenbereich nachweisbar.
5. Abschließend wurden die restlichen Nukleotide von der "tag"-markierten DNA m.H. eines "PCR-Purification Kits" (Diagen GmbH, Hilden, Kat.- Nr. 28106) abgetrennt.

### Schritt 2

**Modifizierung der "tag"-markierten genomischen DNA für die in situ Hybridisierung.**

### Reagen zien, Puffer und Lösungen

- 10x Reaktionspuffer mit 0.5 M Tris-HCl (pH 8,0), 50 mM MgCl₂, 0,5 mg/ml bovinern Serumalbumin (Fraktion V, Kat.- Nr. 735078, Boehringer Mannheim)
- 0,1 M β-Mercaptoethanol.
- 10x Nukleotidstammlösung mit 0,5 mM dATP, 0,5 mM dCTP, 0,5 mM dGTP, 0,125 mM Digoxigenin-11-dUTP (Boehriger Mannheim, Kat.-Nr.1558706), 0,375 mM dTTP.
- Escherichia coli-DNA-Polymerase I (New England Biolabs GmbH, Schwalbach/Taunus, Kat.- Nr. 209L).
- DNase I-Stammlösung: 3 mg in 1 ml 0.15 M NaCl, 50% Glycerol.
- Säulenpuffer: 10 mM Tris-HCI (pH 8,0), 1 mM EDTA, 0.1% SDS.
- Säulen: Sephadex G50 (Medium), 1 ml Tuberkulinspritzen (z.B. "Primo" von Pharmaplast A/S, DK-4970 Rodby), Glaswolle.
- Für Minigel: Agarose; TBE-Puffer; 1kb-Größenmarker (Gibco BRL, Eggenstein, Kat.-Nr. 15615-016); Gelladungspuffer (s.o.)

### Detaillierter Ablauf:

1. 2 µg der "tag"-markierten DNA (s. Schritt 1) wurden zusammen mit 10 µl des 10x Reaktionspuffers, 10 µl β-Mercaptoethanol, 10 µl der Nukleotidstammlösung, 20 U der DNA-Polymerase I and 2 µl einer 1:1000-Verdünnung der DNse-Stammlösung (in Wasser) in ein Reaktionsgefäß pipettiert.
2. Die Modifizierungsreaktion erfolgte bei 15°C für 30-40 min.
3. Das Reaktionsgemisch wurde auf Eis gestellt und ein Aliquot auf die für die in situ Hybridisierung geeignete Fragmentgröße getestet.
4. Die Fragmentlänge der DNA wurde durch Gelelektrophorese bestimmt. 10 µl des Reaktionsanatzes wurden mit 3 µl Gelladungspuffer versetzt und im kochenden Wasserbad für 2-3 min denaturiert. Nach weiteren 3 min auf Eis wurde das Aliquot auf ein 1-2 % Agarose-Minigel zusammen mit dem 1 kb-Größenmarker aufgetragen. Die Auftrennung der DNA-Fragmente erfolgte bei 15 V/cm für 30 min. Nach Anfärbung mit Ethidiumbromid konnte das Gel unter UV-Licht photographiert und die Größe der DNA-Fragmente ermittelt werden.
5. Im optimalen Falle sollten die DNA-Fragmente in einem Größenbereich zwischen 500 und 1000 Basenpaaren liegen. Lag die durchschnittliche Größe der Fragmente oberhalb dieses Bereiches, wurde die verbliebene DNA nochmals mit DNase I inkubiert, bis die optimale Größe der Fragmente erreicht war.
6. Um die DNase zu inaktivieren, wurden dem Ansatz 2 µl 0.5 M EDTA (Endkonzentration 10 mM) und 1 µl 10% SDS (Endkonzentration 0.1%) zugesetzt und der Reaktionsansatz bei 68°C für 10 min erhitzt.
7. Nichtinkorporierte Nukleotide wurden von der DNA-Probe durch Gelfiltration mittels Trennsäulen abgetrennt, die folgendermaßen hergestellt worden waren.
   (a) Eine 1 ml-Tuberkulinspritze wurde zunächst mit Glaswolle bis zur 0.2 ml-Markierung gepackt und dann mit gepuffertem Sephadex G50 bis zur 1 ml-Marke aufgefüllt. Die Säule wurde in ein 15 ml-Gefäß überführt und bei 2000g 6 min. bei Raumtemperatur zentrifugiert.
   (b) Nach Zugabe von je 100 µl des Säulenpuffers wurden die Säulen erneut zentrifugiert. Dieser Schritt wurde dreimal wiederholt.
   (c) Die DNA wurde auf die Säule gegeben, zentrifugiert (2000g 6 min.) und in einem Reaktionsgefäß aufgefangen. Die Endkonzentration dieser DNA-Probe beträgt etwa 20 ng/µl. Sie kann über längere Zeit (Monate bis Jahre) bei -20°C aufbewahrt werden.

### Schritt 3

**In situ Hybridisierung der "tag"-markierten genomischen DNA auf Interphasekerne der Linie Colo320 (HSR).**

### A) Denaturierung der genomischen DNA der Colo320 (HSR) Interphasekerne.

### Reagenzien, Puffer und Lösungen:

- Denaturierungslösung: 70% deionisiertes Formamid (für die Molekularbiologie, Kat.-Nr 112027, Merck, Darmstadt), 2x SSC, 50 mM Natriumphosphat (pH 7 mit 1 M HCl).
- Ethanol (eiskalt): 70%, 90% und 100%.

### Detaillierter Ablauf:

1. Die Denaturierungslösung wurde in eine Küvette gegeben und in einem Wasserbad auf 70°C erhitzt.
2. Das Deckgläser (76 x 26 mm) mit den zu untersuchenden Colo 320(HSR)-Zellkernen wurde genau 2 min in dieser Lösung inkubiert und dann sofort in den kalten 70% Alkohol überführt.
3. Die Deckgläser wurden jeweils 5 min. in 70%, 90% und 100% Ethanol entwässert und dann luftgetrocknet.

### B) Präzipitierung und Denaturierung der "tag"-markierten DNA-Probe für die in situ Hybridisierung.

### Reagenzien, Puffer und Lösungen

- 3 M Natriumacetat, pH 5.2.
- Deionisiertes Formamid (für die Molekularbiologie, Kat.- Nr 112027, Merck, Darmstadt). Die Deionisierung erfolgte durch Rühren des Formamids mit einem Ionenaustauscher (z. B. AG 501-X8 (D) Resin, Kat.- Nr. 142-6425 der Biorad, München).
- 2x Hybridisierungspuffer: 4x SSC, 20% Dextransulfat.

### Detaillierter Ablauf:

1. 1 µg der "tag"-markierten DNA und 50 µg humaner Cotl-DNA (Gibco-BRL, Eggenstein, Kat. Nr. 5279SA) wurden durch Zugabe von 1/20 Volumen 3 M Natriumacetat und 2.5 Volumen 100% Ethanol präzipitiert.
2. Nach Zentrifugation bei 12000 rpm für 10 min und 4°C wurde der Überstand abgetrennt. das Pellet mit 500 µl 70% Ethanol gewaschen, wieder zentrifugiert (12000 rpm, 10 min, 4°C) und dann lyophilisiert.
3. Die präzipitierte DNA wurde in 6 µl deionisiertem Formamid aufgenommen und 30 min bei Raumtemperatur geschüttelt (Vortex).
4. Nach Zugabe von 6 µl 2x Hybridisierungspuffer wurde wiederum 30 min geschüttelt.
5. Die DNA wurde bei 75°C für 5 min. denaturiert, 5 min. auf Eis gestellt und dann 20 - 30 min bei 37°C inkubiert ("preannealing").

### C ) In situ Hybridisierung

### Detaillierter Ablauf:

1. 12 µl des Hybridisierungsmixes mit der denaturierten DNA wurden auf ein großes Deckglas mit den zu untersuchenden Zellkernen gegeben.
2. Ein 18 x 18 mm Deckglas wurde aufgelegt.
3. Das 18 x 18 mm Deckglas wurde mit Flüssigklebstoff abgedichtet und das Präparat in einer feuchten Kammer für 48 h bei 37°C inkubiert.

### D) Waschen und Detektion der hybridisierten Zellkerne

### Reagenzien, Puffer und Lösungen:

- Waschlösung A: 50% Formamid (p. A. Kat.- Nr. 9684, Merck, Darmstadt), 2xSSC.
- Waschlösung B: 0.1x SSC.
- Waschlösung C: 4x SSC, 0.1% Tween 20.
- Waschlösung D: 2x SSC, 0.05% Tween 20.
- "Blocking"-Lösung : 3% BSA, 4x SSC, 0.1% Tween 20.
- Detektionspuffer: 1% BSA, 4x SSC, 0.1% Tween 20.
- Anti-Digoxigenin-Rhodamin, FAB-Fragmente (Kat- Nr. 1207750, Boehringer Mannheim)
- "Antifade"-Lösung: 0.233 g DABCO (1,4-Diazabicyclo-2.2.2-octane), 20 mM Tris-HCl, pH 8.0, 90% Glycerol.
- 4,6-diamino-2-Phenylindol (DAPI)

### Detaillierter Ablauf:

1. Die Waschlösungen A and B wurden in einer Küvette auf 42°C im Wasserbad erwärmt.
2. Der Dichtring aus Flüssigklebstoff wurde vom Deckglas entfernt und dieses dann für 3 x 10 min. in Waschlös. A bei 42°C gewaschen.
3. Anschließend wurde in Waschlösung B 3 x 10 min gewaschen.
4. 200 µl der "Blocking" -Lösung wurden auf das Deckglas pipettiert, wieder ein Deckglas darüber gegeben und 30 min bei 37°C in einer feuchten Kammer inkubiert.
5. Die "Blocking"-Lösung wurde entfernt; stattdessen wurden 200 µl des Detektionspuffers mit 6 µg/ml Rhodamin-konjugiertem Anti-Digoxigenin (Boehringer, Mannheim) auf das Deckglas gegeben und unter einem Deckglas 30 min. bei 37°C in einer feuchten Kammer inkubiert.
6. Das große Deckglas mit den Zellkernen wurde dann in Waschlösung C dreimal je 10 min bei 42°C gewaschen.
7. Anschließend wurde das große Deckglas mit den Zellkernen 20 min in 2x SSC, in welchem 200 ng/ml (DAPI) gelöst waren, inkubiert.
8. Waschen des Deckglases in Waschlösung D für 1-2 min bei Raumtemperatur.
9. Eindecken der hybridisierten Interphasekerne in "Antifade"-Lösung.

### Schritt 4

**Isolierung der hybridisierten Kerne durch Mikromanipulation**

### Geräte:

Mikroskop Axioskop FS (Carl Zeiss, Jena) ausgerüstet für die Fluoreszenzmikroskopie.
De Fonbrune Mikromanipulator (Bachofer, Reutlingen).
De Fonbrune Mikroschmiede (Bachofer, Reutlingen) zur Herstellung von Mikrospitzen. Glasplatten aus B270: Größe 70 x 35 mm, Stärke 6 mm; mit Absatz: Breite 26 mm; Tiefe 4 mm. (Hergestellt durch Berliner Glas KG, Waldkraichburger Straße 5, 12347 Berlin, Best. Nr B 100158 - PO/eh).

### Detaillierter Ablauf:

1. Das große Deckglas mit den zu isolierenden Colo-Zellkernen wurde (mit den Zellen nach unten zeigend) auf die B270 Glasplatte gegeben; der Zwischenraum zwischen Sockelboden und Deckglas (bzw. Zellkernen) wurde mit "Antifade"-Lösung gefüllt.
2. Eine in der Mikroschmiede hergestellte Mikrospitze wurde in den Mikromanipulator eingesetzt. Die Mikrospitze wurde m. H. der Mikroschmiede so ausgezogen, daß sie in einem Winkel von etwa 30° nach oben zeigte.
3. Die Mikrospitze wurde nun in den mit der Antifade-Lösung gefüllten Zwischenraum geschoben, so daß sie von unten an die zu isolierenden Zellkerne reichte.
4. Die Colokerne konnten unter Fluoreszenzbedingungen durch einen geeigneten Filter (Filtersatz Nr. 487915-9901 für Rhodamin oder Filtersatz Nr. 487901-9901 für DAPI, Carl Zeiss, Jena) im Mikroskop sichtbar gemacht werden. Durch Verwendung des Durchlichts war es außerdem möglich, gleichzeitig die Mikrospitze zu erkennen. Auf diese Weise können die Zellkerne einzeln auf die Mikrospitzen "aufzuspießt" werden. (Anmerkung: Da die Zellkerne in der "Antifade"-Lösung nicht brüchig, sondern elastisch sind, können sie als Ganzes isoliert werden).
5. Nachdem jeweils ein Kern mit einer Nadel aufgenommen war, wurde diese am Boden eines mit 20 µl H₂O gefüllten PCR-Röhrchens abgebrochen. Auf diese Weise konnten Populationen mit genau definierter Zellzahl an hybridisierten und nicht-hybridisierten Kernen isoliert werden. (Anmerkung: Die nicht-hybridisierten Zellkerne waren gleich behandelt worden wie die hybridisierten Kerne, jedoch war bei der in situ Hybridisierung keine "tag"-markierte DNA zugegeben worden.

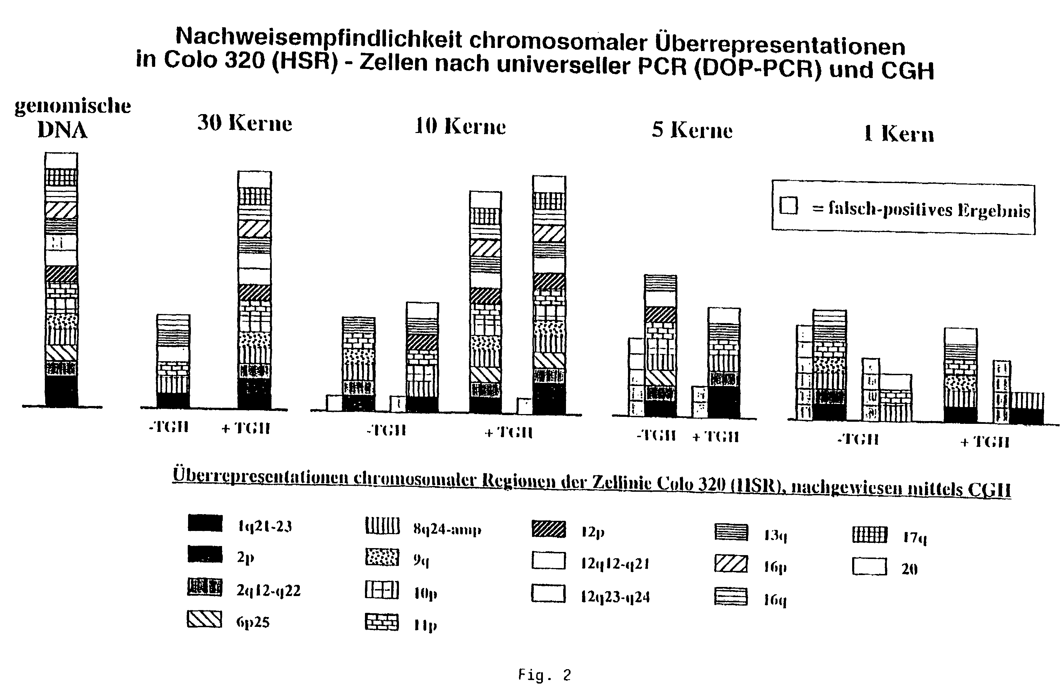

Im Anschluß an diesen Schritt erfolgte die DOP-PCR der isolierten Kerne. Dieses Protokoll ist identisch mit Schritt 1 (Punkte 1-5), jedoch wird außer den isolierten Kernen keine weitere Ausgangs-DNA zugegeben. Das DOP-PCR-Produkt wurde für eine übliche CGH-Analyse verwendet (vgl. Kallioniemi A, et al., Science 258:818, 1992, Kallioniemi O-P, et al., Genes Chromosom Cancer, 10:231, 1994 und Lichter P, et al. in Human Chromosomes, Hrsg. Verma R.S. und Babu A., New York, 1995, S. 191). Es zeigte sich, daß mit dem erfindungsgemäßen Verfahren in 30 bzw. 10 Colo320 (HSR) Zellen zwischen 89% und 94% aller Chromosomen-Überrepräsentationen erkannt wurden, während es ohne das TGH-Verfahren nur zwischen 38% und 44% waren. Ferner liegt der Anteil der falsch-positiven Befunde in der TGH-behandelten Gruppe deutlich niedriger als in der nicht-TGH-behandelten (7 gegenüber 18) (vgl. Fig. 2).

## Patentansprüche

1. Verfahren zum Nachweis numerischer Veränderungen in der DNA von Zeilen, umfassend die folgenden Schritte:
(a) Isollerung von DNA aus Zellen, die keine bekannten numerischen Veränderungen in ihrer DNA aufweisen, und Amplifikation der DNA mittels eines PCR-Verfahrens unter Verwendung von tag-Primen,
(b) In situ Hybridisierung von zu untersuchenden Zellen mit der amplifizierten DNA von (a),
(c) Amplifikation von DNA aus den in situ hybridisierten Zellen von (b) mittels eines PCR-Verfahrens unter Verwendung der tag-Primer von (a), und
(d) Identifizierung numerischer Veränderungen in der amplifizierten DNA von (c).

2. Verfahren nach Anspruch 1, **dadurch gekennzeichnet, daß** die zu untersuchenden Zellen von Tumoren stammen.

3. Verfahren nach Anspruch 1, **dadurch gekennzeichnet, daß** die zu untersuchenden Zellen aus dem Blut von schwangeren Personen stammen.

4. Verfahren nach Anspruch 2 oder 3, **dadurch gekennzeichnet, daß** die zu untersuchenden Zellen solche einer kleinen Zellpopulation oder Einzelzellen sind.

5. Verfahren nach einem der Ansprüche 1 - 4, **dadurch gekennzeichnet, daß** die zu untersuchenden Zellen einen interphase-Kern aufweisen.

6. Verfahren nach einem der Ansprüche 1 - 5, **dadurch gekennzeichnet, daß** die tag-Primer degenerative Primer sind.

7. Verfahren nach einem der Ansprüche 1 - 6, **dadurch gekennzeichnet, daß** die Identifizierung von (d) ein "Comparative Genomic Hybridization (GGH) Verfahren umfaßt.

8. Kit zur Durchführung des Verfahrens nach einem der Ansprüche 1 - 7, umfassend die folgenden Komponenten:
(a) amplifizierte DNA aus Zeilen, die keine bekannten numerischen Veränderungen in ihrer DNA aufweisen, wobei die DNA von tagPrimern flankiert ist,
(b) tag-Primer, und
(c) Hilfsmittel für die Identifizierung numerischer Veränderungen in einer DNA.

## Claims

1. A process for detecting numerical changes in cell DNA, comprising the following steps:
(a) isolation of the DNA from cells, which do not comprise any of the known numerical changes in their DNA and amplification of the DNA by means of a PCR method using tag primers,
(b) *in situ* hybridization of cells under study with the amplified DNA from (a),
(c) amplification of DNA from the *in situ* hybridized cells from (b) by means of a PCR method using the tag primers from (a), and
(d) identification of numerical changes in the amplified DNA from (c).

2. The process according to claim 1, **characterized in that** the cells under study originate from tumors.

3. The process according to claim 1, **characterized in that** the cells under study originate from the blood of pregnant persons.

4. The process according to claim 2 or 3, **characterized in that** the cells under study are those of a small cell population or single cells.

5. The process according to any one of claims 1 to 4, **characterized in that** the cells under study have an interphase nucleus.

6. The process according to any one of claims 1 to 5, **characterized in that** the tag primers are degenerative primers.

7. The process according to any one of claims 1 to 6, **characterized in that** the identification from (d) comprises a "Comparative Genomic Hybridization (CGH) method".

8. A kit for carrying out the process according to any one of claims 1 to 7, comprising the following components:
(a) amplified DNA from cells, which do not comprise any of the known numerical changes in their DNA, and the DNA being flanked by tag primers,
(b) tag primers, and
(c) auxiliary agents for identifying numerical changes in DNA.

## Revendications

1. Procédé de détection de variations numériques dans l'ADN de cellules, comprenant les étapes suivantes :
(a) isolement d'ADN de cellules dont l'ADN ne présente pas de variations numériques connues, et amplification de l'ADN par une méthode de réaction en chaîne de la polymérase en présence d'amorces tag,
(b) hybridation in situ de cellules à examiner avec l'ADN amplifie de (a),
(c) amplificalion d'ADN des cellulcs hybridées in situ de (b) par une méthode de réaction en chaine de la polymérase en présence des amorces tag de (a), et
(d) identification de variations numériques dans l'ADN amplifié de (c).

2. Procédé suivant la revendication 1, **caractérisé en ce que** les cellules à examiner proviennent de tumeurs.

3. Procédé suivant la revendication 1, **caractérisé en ce que** les cellules à examiner proviennent du sang de femme enceinte.

4. Procédé suivant la revendication 2 ou 3, **caractérisé en ce que** les cellules à examiner sont celles d'une petite population de cellules ou sont des cellules individuelles.

5. Procédé suivant l'une des revendications 1 à 4, **caractérisé en ce que** les cellules à examiner présentent un noyau d'interphase.

6. Procédé suivant l'une des revendications 1 à 5, **caractérisé en ce que** les amorces tag sont des amorces dégénératives.

7. Procédé suivant l'une des revendications 1 à 6, **caractérisé en ce que** l'identification de (d) comprend une opération de « comparative genomic hybridization » (CGH).

8. Nécessaire destiné à la mise en oeuvre du procédé suivant l'une des revendications 1 à 7, comprenant les composants suivants :
(a) un ADN amplifié de cellules dont l'ADN ne présente pas de variations numériques connues, cet ADN étant flanqué d'amorces tag,
(b) des amorces tag, et
(c) des moyens auxiliaires pour l'identification de variations numériques dans un ADN,
